# EUROPÄISCHE PATENTSCHRIFT

(11) **EP 0 983 495 B1**
(45) Veröffentlichungstag und Bekanntmachung des Hinweises auf die Patenterteilung: **02.01.2002**
(21) Anmeldenummer: 98933534.4
(22) Anmeldetag: 14.05.1998
(51) Int. Cl.: G01N 3/40, G01N 33/15

(54) **VERFAHREN UND VORRICHTUNG ZUR DURCHFÜHRUNG EINES HÄRTETESTS AN PRÜFKÖRPERN, INSBESONDERE TABLETTEN ODER PILLEN**
METHOD AND DEVICE FOR CONDUCTING A HARDNESS TEST ON TEST SPECIMENS, SPECIALLY TABLETS OR PILLS
PROCEDE ET DISPOSITIF POUR REALISER UN TEST DE DURETE SUR DES EPROUVETTES, NOTAMMENT DES COMPRIMES OU DES PILULES

(30) Priorität: 23.05.1997 DE 19721656; 09.06.1997 DE 19724121; 07.10.1997 DE 19744227
(43) Veröffentlichungstag der Anmeldung: 08.03.2000
(73) Patentinhaber: Krämer, Norbert, 64291 Darmstadt (DE)
(72) Erfinder: Krämer, Norbert, 64291 Darmstadt (DE)
(74) Vertreter: Mierswa, Klaus
(86) Internationale Anmeldenummer: DE9801345
(87) Internationale Veröffentlichungsnummer: WO9853298

(56) Entgegenhaltungen:
- DE-A- 4 241 985
- US-A- 4 641 534
- US-A- 4 884 463
- US-A- 5 555 768

## Beschreibung

### Technisches Gebiet:

Die Erfindung betrifft ein Verfahren und eine Vorrichtung zur Durchführung eines Härtetests an Prüfkörpern, inbesondere Tabletten oder Pillen gemäß den Oberbegriffen der Ansprüche 1 bzw. 5.

### Stand der Technik:

Im Rahmen der Qualitätskontrolle bei der Fertigung von Tabletten werden physikalische Eigenschaften von Tabletten wie z.B. Gewicht, Abmessungen, Zerfallszeit in einem Medium und Härte bestimmt. Dazu sind Tablettenprüfsysteme entwickelt worden, welche jeweils eines Mehrzahl von Tabletten aus einem Produktionszyklus auf diese Eigenschaften hin untersuchen. Tabletten einer Charge werden auf einem Vorratsbehälter vereinzelt und z.B. auf einem Transportband von einer Meßstation zur nächsten befördert.

Die Härte des Prüfkörpers wird üblicherweise in einer Kraftmeßdose gemessen, welche als wesentliche Bestandteile einen Druckkolben und ein Gegenlager aufweist. Zur Durchführung des Härtetests wird der Prüfkörper, also die Tablette, in den Bereich zwischen Druckkolben und Gegenlager befördert, wobei die Tablette vorzugsweise das Gegenlager berührt. Der Druckkolben wird nun mittels eines Schrittmotors gegen das Gegenlager und die vor diesem liegende Tablette gefahren. Die vom Druckkolben mit jedem Schritt des Motors ausgeübte Kraft wird gemessen und aufgezeichnet. Diese Kraft ist konstant und sehr klein, solange der Druckkolben die Tablette nicht berührt oder diese ohne den Gegendruck des Gegenlagers über den Prüftisch schiebt. Wenn der Druckkolben die Tablette berührt und gegen das Gegenlager drückt, steigt die von ihm ausgeübte Kraft mit jedem Schritt des Schrittmotors so lange an, bis die Tablette zerbricht. Die zum Zerbbrechen der Tablette aufgewendete Kraft wird aufgezeichnet und dient als Maß für die Härte der Tablette. Das plötzliche Abfallen der vom Druckkolben aufgewendeten Kraft beim Zerbrechen des Prüfkörpers dient als Abbruchbedingung für das Beenden der Messung. Der Druckkolben wird in seine Ausgangsposition zurückgefahren, und die nächste Tablette kann geprüft werden.

Um aussagekräftige, reproduzierbare Härtewerte zu liefern, sollte die Messung der Härte, d. h. das Zerdrücken der Tablette zwischen Druckkolben und Gegenlager, stets entlang einer definierten Achse erfolgen. Dies ist bei nicht symmetrisch geformten Tabletten schon allein deswegen zu beachten, weil oft mit der Härtemessung gleichzeitig auch eine Dickenmessung durchgeführt wird. Die Stellung des Druckkolbens beim Abbruch der Härtemessung dient dabei als Maß für die Dicke. Weiterhin können derartige Tabletten entlang verschiedener Achsen durchaus verschiedene Härtewerte besitzen.

Bei länglich ellipsoid geformten Tabletten gelingt die Ausrichtung während des Transports auf dem Prüftisch beispielsweise passiv mittels eines Schiebers, welcher die Tablette quer zur Transportrichtung ausrichtet. Unregelmäßig geformte Tabletten lassen sich jedoch nur schwer auf die Weise passiv ausrichten. Bei der Härtemessung derartiger Tabletten muß daher in den herhömmlichen Prüfsystemen mit größeren Meßfehlern gerechnet werden.

Zur Vermeidung solcher Meßfehler ist es bekannt, die Tablette aktiv auszurichten, so daß ihre Vorzugsrichtung, entlang derer die Härte gemessen werden soll, mit der Bewegungsrichtung des Druckkolbens gegen das Gegenlager übereinstimmt. Die Position und Ausrichtung der Tablette auf einem Prüftisch zwischen Druckkolben und Gegenlager wird dazu mit einer Kamera aufgezeichnet und über Bildverarbeitung ausgewertet. Aus den Bilddaten läßt sich ermitteln, um wieviel Grad die Vorzugsachse des Prüfkörpers von der Bewegungsrichtung von Druckkolben und Gegenlager abweicht. Diese Abweichung kann nun durch geeignete Maßnahmen korrigiert werden.

Dazu ist eine Vorrichtung bekannt, bei welcher sich die Tablette auf einem drehbaren Prüftisch befindet. Die Kraftmeßdose, insbesondere Druckkolben und Gegenlager sind ortsfest. Damit ist die Richtung der Härtemessung im Raum festgelegt. Der Prüftisch wird nun - kontrolliert durch das Kamerasignal - solange gedreht, bis die Vorzugsachse des Prüfkörpers mit der Bewegungsrichtung des Druckkolbens gegen das Gegenlager übereinstimmt. So gelingt eine Härtemessung in Richtung der Vorzugsachse des Prüfkörpers. US-A-5 555 768 beschreibt ein Verfahren bzw. eine Vorrichtung zur Durchführung eines Härtetests mit einem nur vertikal beweglichen Druckkolben. Der Prüftisch wird gedreht, um den Prüfkörper mit dem Druckkolben auszurichten.

Nachteilig an derartigen Vorrichtungen ist allerdings, daß die Zeit zum Ausrichten des Prüfkörpers in Bewegungsrichtung des Druckkolbens oft sehr lang ist. Die Ursache dafür liegt darin, daß die Bewegung des Prüfkörpers durch eine Drehung des Prüftisches aufgrund der geringen Reibung zwischen Prüfkörper und Prüftisch nur schwer kontrollierbar ist.

Dies kann zur Folge haben, daß der Prüfkörper der Drehbewegung des Prüftisches oft gar nicht oder nur unvollständig folgt. Weiterhin dreht sich der einmal in Gang gesetzte Prüfkörper auch bei stillstehendem Prüftisch weiter. Nach jeder Drehung des Prüftisches muß daher die Position und Vorzugsachse des Prüfkörpers neu ermittelt werden und ggf. der Prüftisch korrigierend gedreht werden. Dies wird so oft wiederholt, bis die Vorzugsachse des Prüfkörpers innerhalb bestimmter Toleranzen mit der Bewegungsrichtung des Druckkolbens übereinstimmt. Erst dann kann der Härtetest durchgeführt werden. Der Nachteil dieser aktiven Anpassung der Vorzugsachse des Prüfkörpers liegt daher in oftmals mehreren korrigiernden Arbeitsschritten, die zur Anpassung nötig sind, also in einem hohen Rechenaufwand.

### Technische Aufgabe:

Der Erfindung liegt daher die Aufgabe zugrunde, ein Verfahren und eine Vorrichtung zur Durchführung eines Härtetests bei Prüfkörpern, insbesondere Tabletten oder Pillen, zu entwickeln, bei welcher das Ausrichten des Prüfkörpers relativ zu Druckkolben und Gegenlager in kurzer Zeit und mit wenig Rechenaufwand zu bewerkstelligen ist.

### Offenbarung der Erfindung und ihrer Vorteile:

Die Lösung der Aufgabe besteht erfindungsgemäß bei einem Verfahren zur Durchführung eines Härtetests an Prüfkörpern, wobei der Prüfkörper auf einem Prüftisch zwischen einen Druckkolben und ein Gegenlager befördert und die Härte des Prüfkörpers durch das Gegeneinanderdrücken von Druckkolben und Gegenlager gemessen wird, bei welchem auf optischem oder akustischem Weg die räumliche Lage einer Vorzugsachse des Prüfkörpers ermittelt wird. Die Vorzugsachse ist dabei diejenige Achse, entlang derer der Härtetest durchgeführt werden soll, z.B. die Längsachse des Prüflings. Weiterhin werden Korrekturdaten ermittelt, die von diesen Lagedaten und von der Position von Druckkolben und Gegenlager abhängig sind, wobei die Position von Druckkolben und Gegenlager auf dem Weg wie die Lage des Prüflings oder elektronisch aus der Stellung von zur Bewegung dieser Elemente verwendeten Steuerelementen oder aus der vorangegangenen Messung ermittelt werden kann. Unter Zuhilfenahme dieser Korrekturdaten werden Druckkolben und Gegenlager aufgrund so relativ zum in seiner Lage auf dem Prüftisch unveränderten Prüfkörper verfahren, daß die Prüfrichtung, d.h. die Richtung des Gegeneinanderdrückens von Druckkolben und Gegenlager, mit der Richtung der Vorzugsachse übereinstimmt und das Gegenlager in unmittelbare Nähe des Prüfkörpers gebracht wird. Die Härte des Prüfkörpers wird daraufhin durch Herandrücken des Druckkolbens gegen den Prüfkörper bei unveränderter Position des Gegenlagers in bekannter Weise bestimmt

Die Lösung der Aufgabe besteht weiterhin bei einer Vorrichtung zur Durchführung eines Härtetests an Prüfkörpern mit einem Prüftisch zur Aufnahme des Prüfkörpers sowie einem Druckkolben und einem Gegenlager, die oberhalb des Prüftischs angeordnet und gegeneinander verfahrbar sind, wobei sich der Prüfkörper im Anwendungsfall zwischen Druckkolben und Gegenlager befindet und die beim Gegeneinanderdrücken von Druckkolben und Gegenlager aufgewendete Kraft oder eine dazu proportionale Größe mittels einer Kraftmeßvorrichtung meßbar und die Härte des Prüfkörpers daraus bestimmbar ist. Mittels wenigstens eines Steuerelements sind Druckkolben und Gegenlager in der Ebene des Prüftischs in zwei Raumrichtungen verfahrbar sind, vorzugsweise sind sie auch senkrecht zur Prüftischebene verfahrbar. Es ist eine Vorrichtung zur Lageerkennung des auf dem Prüftisch liegenden Prüfkörpers vorgesehen, welche ein Bild der Meßstelle auf optischem oder akustischem Weg aufzuzeichnen und aus der räumlichen Lage von Druckkolben und Gegenlager sowie einer Vorzugsachse des Prüfkörpers Korrekturdaten zu ermitteln imstande ist, die als Ausgangssignal abgegeben werden. Die Vorrichtung zur Lageerkennung besteht beispielsweise aus einer optischen oder akustischen Sensoreinheit, z.B. auch eine Kamera oder ein Sensorarray, auch aus Berührungssensoren, welche mit einer Datenverarbeitungsanlage gekoppelt ist. Die Signale der die Meßstelle optisch oder akustisch abtastenden Einheit werden aufgezeichnet, in ein Bild umgewandelt und beispielsweise mittels Bildverarbeitung zur Ermittlung der Lageinformationen und schließlich der Korrekturdaten ausgewertet. Das Ausgangssignal der Vorrichtung zur Lageerkennung dient zur Ansteuerung des Steuerelements bzw. der Steuerelemente derart, daß die Prüfrichtung mit der Richtung der im Raum festliegenden Vorzugsachse des Prüfkörpers in Übereinstimmung gebracht wird.

Vorzugsweise werden diese Korrekturdaten zur Steuerung des Anpassungsprozesses mehrmals während Anpassung der Lage von Druckkolben und Gegenlager an die Lage des Prüfkörpers ermittelt.

Weitere vorteilhafte Ausgestaltungen der Erfindung sind in den übrigen Unteransprüchen gekennzeichnet.

Die erfindungsgemäße Vorrichtung bzw. das Verfahren zur Durchführung eines Härtetests an Prüfkörpern hat insbesondere den Vorteil, daß die Lage des Prüfkörpers im Raum vor und während des Härtetests unverändert bleibt. Dadurch werden aktive Bewegungen des Prüfkörpers, z.B. durch Drehung des Prüftisches, vorteilhaft vermieden. Diese sind schwer kontrollierbar, da der Prüfkörper nicht mit dem Prüftisch oder eines anderen Elementes der Vorrichtung gekoppelt ist. Lage und Ausrichtung des Prüfkörpers müssen nur einmalig mittels der Vorrichtung zur Lageerkennung ermittelt werden. Entsprechend diesen Daten werden Druckkolben und Gegenlager, vorzugsweise rechnergesteuert, mittels der Steuerelemente so bewegt, daß ihre Ausrichtung und Bewegungsrichtung beim Gegeneinanderdrücken mit der Vorzugsachse des Prüfkörpers übereinstimmt. Die Bewegung von Druckkolben und Gegenlager ist dabei leicht kontrollierbar und nicht zufallsbestimmt, da Druckkolben und Gegenlager direkt oder indirekt mit den Steuerelementen, wie z.B. Motoren, verbunden sind.

In einem einzigen Arbeitsschritt kann daher die Bewegungsrichtung von Druckkolben relativ zum Gegenlager mit der Richtung der Vorzugsachse des Prüfköpers in Übereinstimmung gebracht werden. In einem zweiten Arbeitsschritt wird vorzugsweise das Gegenlager an den Prüfkörper herangefahren, so daß die Frontfläche des Gegenlagers den Prüfkörper berührt, diesen jedoch nicht bewegt. Diese Ausgangssituation ist zur Durchführung des Härtetests notwendig, da der Druckkolben die Tablette ohne den Gegendruck des Gegenlagers verdrehen würde. Der Druckkolben wird nun an den Prüfkörper herangefahren und die Härte der Tablette in bekannter Weise gemessen.

Die Relativbewegung von Tablette zu Druckkolben bzw. Gegenlager ist bei der erfindungsgemäßen Vorrichtung stets genau kontrollierbar. Daher ist im Prinzip nur eine einmalige Auswertung der Ausgangssignale der Vorrichtung zur Lageerkennung zur Bestimmung der Position und Ausrichtung des Prüfkörpers notwendig. Die Bilddaten werden jedoch vorzugsweise mehrfach - insbesondere vor und nach dem Drehen von Druckkolben und Gegenlager - ausgewertet, um zu überprüfen, ob die Ausrichtung der Achsen erfolgreich durchgeführt wurde.

Zur Anpassung der Achsen wird beispielsweise der Winkel zwischen der Vorzugsachse des Prüfkörpers und der Prüfrichtung ermittelt und Druckkolben und Gegenlager werden gemeinsam um diesen Winkel gedreht. Anschließend wird der Abstand des Gegenlagers zum Prüfkörper ermittelt und das Gegenlager um diesen Abstand derart verfahren, daß es sich in unmittelbarer Nähe des Prüfkörpers befindet. Daraufhin wird der Härtetest ausgeführt.

Weiterhin kann die Bilddatenauswertung auch kontinuierlich erfolgen, z.B. nach jedem Schritt des drehenden Steuerelements. Dann kann u.U. auf eine vorherige Bestimmung des Drehwinkels verzichtet und stattdessen die Übereinstimmung der Achse des Prüfkörpers und der Verbindungsachse von Druckkolben und Gegenlager als Abbruchbedingung für eine weitere Drehung verwendet werden.

Da die Bewegung von Druckkolben und Gegenlager gut kontrollierbar ist, gelingt in jedem Fall ein schnelles Ausrichten der jeweiligen Achse und ein Nachkorrigieren der Lage der Achsen wird vermieden.

Bei der erfindungsgemäßen Vorrichtung befinden sich Druckkolben und Gegenlager vorzugsweise höchstens wenige Millimeter oberhalb der Tischfläche des Prüftisches. Sie können daher mit wenig oder gar keiner Reibung über diese Tischfläche gleiten bzw. gefahren werden. Der Abstand zwischen der Tischfläche des Prüftisches und der Unterseite von Druckkolben und Gegenlager ist stets konstant, und die Unterseite von Druckkolben und Gegenlager verläuft parallel zur Tischfläche.

Vorzugsweise sind Druckkolben und Gegenlager über einen Haltebügel miteinander verbunden. Dabei sind sie derart über ein oder mehrere Steuerelemente, z.B. Schrittmotoren, am Haltebügel befestigt, daß der Abstand ihrer jeweiligen Frontflächen veränderlich ist. Es ist möglich, daß sowohl Druckkolben als auch Gegenlager mittels Steuerelementen relativ zum Haltebügel beweglich sind oder nur der Druckkolben. Diese Steuerelemente können zum Heranfahren des Gegenlagers direkt an den Prüfkörper und zur Durchzuführung des Härtetests durch die Bewegung des Druckkolbens dienen. Dazu kann der Haltebügel auch als Ganzes verschiebbar sein.

Vorzugsweise ist der Haltebügel um eine senkrecht zum Prüftisch laufende Drehachse mindestens um einen Winkel von nahezu 180° drehbar. Durch die Drehung des Haltebügels wird die Verbindungsachse von Druckkolben und Gegenlager, entlang derer der Druckkolben gegen das Gegenlager zur Durchführung des Härtetests gedrückt wird, mit der Vorzugsachse des Prüfkörpers in Übereinstimmung gebracht.

Vorzugsweise ist der gesamte Haltebügel in zwei Raumrichtungen parallel zum Prüftisch beweglich. Zur Durchführung des Härtetests wird damit zunächst durch Drehung des Haltebügels die Verbindungsachse zwischen Druckkolben und Gegenlager mit der Vorzugsachse des Prüfkörpers in Übereinstimmung gebracht. Dann wird der gesamte Haltebügel so verfahren, daß die Frontfläche des Gegenlagers den Prüfkörper gerade noch nicht berührt, d.h. sich in einem Abstand von 0,1 bis 1 mm befindet. Ist der Haltebügel parallel zum Prüftisch beweglich, kann somit auch auf ein Steuerelement zur Bewegung des Gegenlagers relativ zum Haltebügel verzichtet werden, so daß nur das Steuerelement zum Heranfahren des Druckkolbens an den Prüfkörper notwendig ist.

In einer weiteren vorteilhaften Ausgestaltung der Erfindung ist der Haltebügel senkrecht zum Prüftisch beweglich. Dies ermöglicht ein Hochfahren des Haltebügels mitsamt des daran gehalterten Druckkolbens und Gegenlagers, z.B. zum leichteren Reinigen des Prüftisches.

Zur Ermöglichung der Bewegung des Haltebügels ist der Haltebügel vorzugsweise mit einem Haltearm gehaltert. Der Haltearm kann den Haltebügel z.B. kranartig über dem Prüftisch haltern. Weiterhin ist zur Erhöhung der Stabilität der Anordnung ein die Meßstelle brückenartig überspannender Haltearm vorteilhaft.

Eine weitere vorteilhafte Realisierung einer über dem Prüftisch frei beweglichen Kraftmeßdose bzw. eines Druckkolbens mit Gegenlager ist z.B. die Befestigung von Druckkolben und Gegenlager an einem um die Meßstelle parallel zur Tischebene herumlaufenden Ring. Druckkolben und Gegenlager werden dann mittels Schrittmotoren in radialer Richtung gegen den Prüfkörper bewegt, wobei wiederum zuerst das Gegenlager an den Prüfkörper herangefahren wird und anschließend der Härtetest durch Gegendrücken des Druckkolbens durchgeführt wird. Zur Einstellung der Prüfrichtung wird entweder der gesamte Ring um eine zur Tischfläche senkrecht verlaufende Achse - vorzugsweise durch den Ringmittelpunkt - gedreht oder Druckkolben und Gegenlager werden innerhalb des Rings verfahren, wobei sie sich jedoch stets gegenüber stehen.

Die Vorrichtung zur Lageerkennung arbeitet vorzugsweise optisch, oder elektronisch, ist beispielsweise eine Kamera, insbesondere CCD-Kamera, oder eine Vorrichtung zur Abtastung eines Objekts mittels Laserstrahlen oder mittels Berührungssensoren. Weiterhin ist auch ein akustisches Meßprinzip zur Lageerkennung möglich, z.B. mittels Ultraschall.

In einer weiteren Ausgestaltung der Erfindung ist die Vorrichtung zur Lagebestimmung eine Matrix aus optischen oder Berührungssensoren, die in den Prüftisch wenigstens im Bereich der Meßstelle eingebracht ist und Konturen auf dem Prüftisch liegender Prüfkörper zu erkennen imstande ist. Ein derartiger Aufbau ist besonders platzsparend und unempfindlich gegen Vibrationen. Diese Matrix ist beispielsweise eine CCD-Matrix oder ein Diodenarray. Zum Schutz der Matrix kann die Matrix durch eine Scheibe aus transparentem Material, z.B. Glas oder Plexiglas, abgedeckt sein.

In einer weiteren Ausgestaltung der Vorrichtung ist der Prüftisch wenigstens teilweise optisch transparent, wobei die Kamera unterhalb des Prüftisches angeordnet ist. Der Prüftisch besteht in diesem Fall z.B. ganz oder mindestens im Bereich der Meßstelle aus Plexiglas. Ein derartiger Aufbau ist platzsparend, da auf Halterungen für die Kamera oberhalb der Meßstelle verzichtet werden kann.

Zur Vereinfachung der Auswertung des Kamerasignals mittels Bildverarbeitung ist es von Vorteil, wenn der Prüftisch eine Markierung, insbesondere ein Raster aufweist.

Kurzbeschreibung der Zeichnung, wobei zeigen:
- Figuren 1,2: eine Härtetestvorrichtung in Seitenansicht in zwei Positionen des Haltebügels
- Figuren 3-5: eine Aufsicht auf den Prüftisch einer Härtetestvorrichtung zur Darstellung der einzelnen Arbeitsschritte bei der Durchführung des Härtetests
- Figuren 6,7: eine Aufsicht auf eine Härtetestvorrichtung mit einem Ring als Halterung
- Figuren 8,9: eine Aufsicht auf den Prüftisch einer Härtetestvorrichtung mit einer Sensormatrix zur Lageerkennung
- Figur 10: eine Härtetestvorrichtung mit teilweise transparentem Prüftisch und Kameraanordnung unterhalb desselben in Seitenansicht

### Bevorzugte Ausführungsformen:

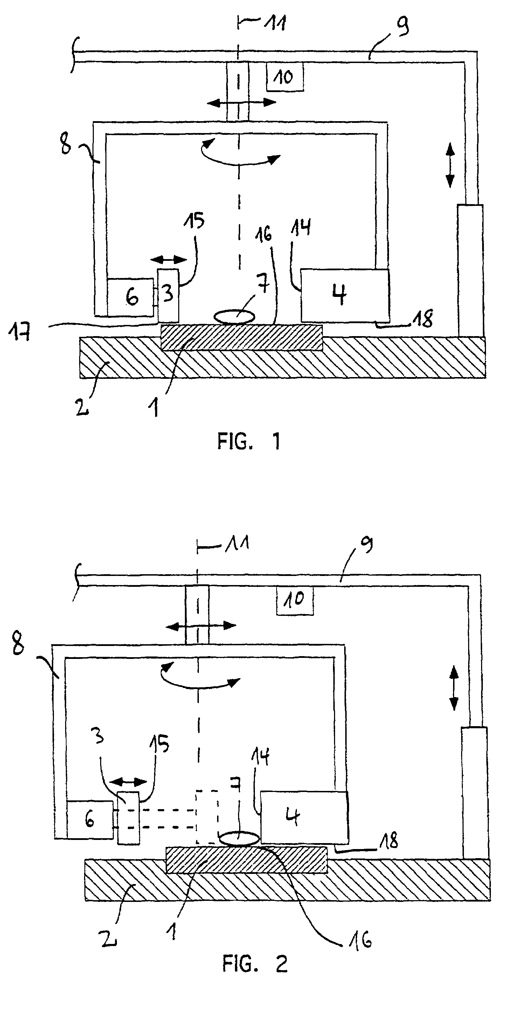

Die Figuren 1 und 2 zeigen die Seitenansicht einer erfindungsgemäßen Vorrichtung zur Durchführung eines Härtetests in zwei verschiedenen Positionen des Haltebügels 8. Ein ellipsoid geformter Prüfkörper 7, z.B. eine Tablette, befindet sich auf einem Prüftisch 1. Der Prüftisch 1 ist in einen Untertisch 2 eingebettet. Der Prüftisch 1 kann zum Transport des Prüfkörpers entweder relativ zum Untertisch 2 bewegt werden, oder der Transport des Prüfkörpers erfolgt mit geeigneten, hier nicht dargestellten Schiebern.

Seitlich oberhalb des Prüftisches 1 befinden sich Druckkolben 3 und Gegenlager 4, wobei der Druckkolben über ein Steuerelement 6 mit einem Haltebügel 8 verbunden ist. Das Steuerelement 6 ist beispielsweise ein Schrittmotor. Mittels des Steuerelements 6 kann der Druckkolben 3 auf das Gegenlager 4 zugeschoben und derart bewegt werden, daß der Abstand der Frontflächen 14 bzw. 15 von Druckkolben 3 und Gegenlager 4 veränderlich ist, wobei die Frontflächen jedoch stets parallel zueinander stehen. Das Gegenlager 4 ist entsprechend den derzeitigen Richtlinien für derartige Prüfgeräte, z.B. nach USP, fest am Haltebügel 8 befestigt. Dadurch werden Ungenauigkeiten in der Bestimmung der Härte des Prüfkörpers gegenüber dem Fall, in dem sowohl Druckkolben 3 als auch Gegenlager 4 unanbhängig voneinander beweglich sind, vermindert, da ein starr mit dem Haltebügel verbundenes Gegenlager dem Druck des Druckkolbens nicht nachgeben und somit die Messung verfälschen kann.

Der Haltebügel 8 ist an einem Haltearm 9 befestigt. Der Haltearm 9 ist derart am Untertisch 2 angeordnet, daß er senkrecht zur Tischfläche 16 verfahren werden kann. Dadurch ist der Haltebügel 8 mitsamt den daran angeordneten Elementen senkrecht zur Tischfläche beweglich. Der Haltebügel 8 ist derart mit dem Haltearm verbunden, daß er um eine senkrecht zur Tischfläche 16 verlaufenden Drehachse 11 gedreht werden kann. Die Drehung erfolgt rechnergesteuert mit einem Motor. Der Drehwinkel beträgt dabei wenigstens nahezu 180° oder mehr. Durch die Drehung des Haltebügels 8 werden Druckkolben 3 und Gegenlager 4 so ausgerichtet, daß ihre Bewegungsrichtung beim Gegeneinanderdrücken, die Prüfrichtung, mit der Richtung der Vorzugsachse des Prüfkörpers 7 übereinstimmt. Dies ist in den Figuren 2 bis 4 näher dargestellt.

Der Haltebügel 8 ist weiterhin derart mit dem Haltearm 9 verbunden, daß er mitsamt Druckkolben 3 und Gegenlager 4, welche an ihm befestigt sind, parallel zur Tischfläche verfahrbar ist. Diese Bewegung erfolgt ebenfalls rechnergesteuert durch einen Motor. Vorzugsweise wird bei der Durchführung des Härtetests zunächst die Prüfrichtung mit der Vorzugsrichtung des Prüfkörpers in Übereinstimmung gebracht und danach der Haltebügel derart verfahren, daß die Frontfläche 14 des Gegenlagers 4 den Prüfkörper berührt. Figur 1 zeigt die Ausgangsposition der erfindungsgemäßen Vorrichtung, Figur 2 die Lage von Druckkolben 3 und Gegenlager 4 nach Verschieben des Haltebügels parallel zum Prüftisch. In Figur 2 gestrichelt dargestellt ist die Position des Druckkolbens bei der Messung der Härte selbst, d.h. beim tatsächlichen Zerdrücken des Prüfkörpers 7 zwischen Druckkolben 3 und Gegenlager 4.

Die Bewegung von Druckkolben 3 und Gegenlager 4 erfolgt parallel zur Tischfläche 16 des Prüftisches. Der Abstand zwischen Tischfläche 16 und den Unterseiten 17 und 18 von Druckkolben 3 bzw. Gegenlager 4, ist dabei konstant und beträgt höchstens wenige Millimeter. Druckkolben 3 und Gegenlager 4 können auch auf dem Prüftisch aufliegen. Starke Reibung zwischen der Tischfläche und den aufliegenden bzw. darüber angeordneten Elementen ist dabei zu vermeiden.

Oberhalb des Prüftisches 1 ist eine Kamera 10 angeordnet, welche ein Bild des Prüftisches mitsamt des darauf aufliegenden Prüfkörpers 7 und ggf. von Druckkolben und Gegenlager aufzeichnet. Die Kamera ist beispielsweise eine CCD-Kamera. Die Bilddaten der Kamera 10 werden einer hier nicht dargestellten Rechenanlage zugeführt, welche über Bildverarbeitung die Vorzugsachse des Prüfkörpers ermittelt. Durch Vergleich mit der Position von Druckkolben und Gegenlager, welche entweder ein zuvor eingestellter Startwert, entsprechend der Ruheposition dieser Elemente, ist oder auch aus den Kamerasignalen ermittelt wird, wird der Drehwinkel berechnet, mit welchem der Haltebügel 8 um die Drehachse 11 gedreht werden muß.

Nach Durchführung der Drehung kann erneut ein Kamerabild zur Überprüfung der Ausrichtung von Druckkolben und Gegenlager ausgewertet werden.

Die Drehung des Haltebügels 8 um die Drehachse 11 erfolgt mittels eines Motors, welcher z.B. am Haltearm 9 angeordnet ist. Die Ansteuerung des Motors erfolgt beispielsweise über denselben Rechner, welcher auch die Bilddaten der Kamer 10 auswertet.

Neben der Position und Ausrichtung des Prüfkörpers 7 kann mit der Kamera 10 auch die Höhe bzw. Breite des Prüfkörpers gemessen werden. Damit können dementsprechende Meßstationen zur Prüfung der physikalischen Eigenschaften eingespart werden.

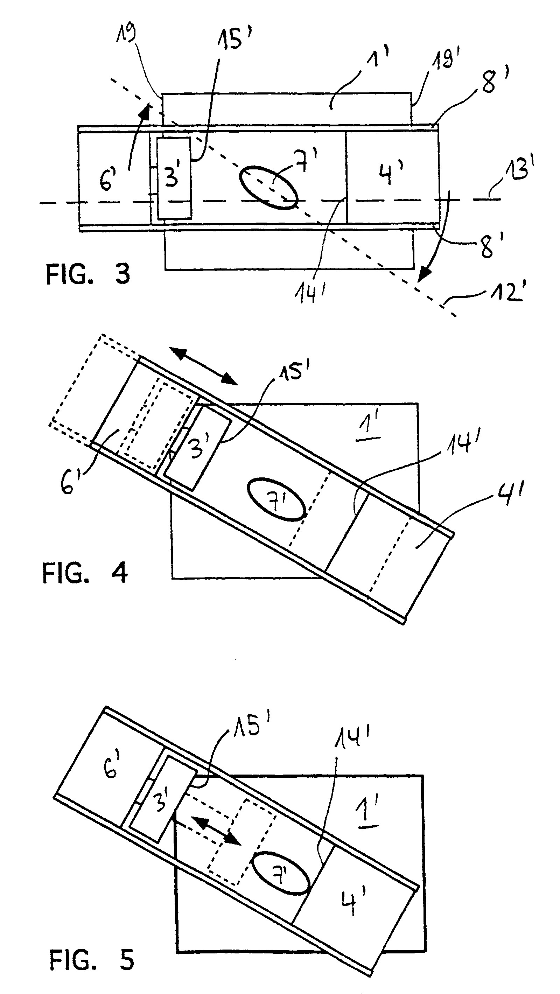

Die Figuren 3 bis 5 zeigen die Aufsicht auf den Prüftisch 1' einer erfindungsgemäßen Vorrichtung zur Darstellung der einzelnen Arbeitsschritte bei Durchführung eines Härtetests.

In Figur 3 ist die Ausgangssituation gezeigt. Ein Prüfkörper 7' befindet sich in beliebiger Ausrichtung auf dem Prüftisch 1' zwischen Druckkolben 3' und Gegenlager 4', welche mit einem Haltebügel 8', bestehend aus zwei Schienen, verbunden sind. Gegenlager 4' und das Steuerelement 6' zur Bewegung des Druckkolbens 3' sind starr an diesen Schienen befestigt. Der Druckkolben 3' ist mittels des Steuerelements 6' derart in Richtung der Schienen beweglich, daß der Abstand zwischen den Frontflächen 15' bzw. 14' von Druckkolben 3' und Gegenlager 4' veränderlich ist. Die Richtung des Gegeneinanderdrückens von Druckkolben 3' und Gegenlager 4' ist die momentane Prüfrichtung 13'. Die Richtung, entlang derer der Härtetest tatsächlich durchgeführt werden soll, ist durch die Vorzugsachse 12' des Prüfkörpers 7' gegeben, welche gegen die momentane Prüfrichtung 13' versetzt ist. In der dargestellten relativen Lage von Druckkolben, Gegenlager und Prüfkörper würde die Härte der Tablette ohne korrigierende Maßnahmen wie das Drehen des Haltebügels 8' also nicht entlang ihrer Vorzugsrichtung, sondern entlang der Richtung 13' gemessen. Der Winkel zwischen den Richtungen 12' und 13' ist zufällig und beträgt hier etwa 30 °. Zweck der Vorrichtung ist es, die Richtungen 12' und 13' in Übereinstimmung zu bringen, indem durch Drehung von Druckkolben und Gegenlager die Prüfrichtung 13' an die Vorzugsrichtung 12' angepaßt wird.

Druckkolben und Gegenlager befinden sich in Figur 3 in ihrer Ausgangs-und Ruheposition derart, daß ihre Frontflächen 14' und 15' parallel zur seitlichen Begrenzung 19,19' des Prüftisches verlaufen. Prinzipiell ist jedoch jede beliebige Ausgangsposition möglich. Druckkolben 3' und Gegenlager 4' sind am Haltebügel 8' gehaltert. Mittels dies Steuerelements 6' kann der Druckkolben in Prüfrichtung 13' bewegt werden.

In einem ersten Arbeitsschritt wird die Vorzugsachse 12' des Prüfkörpers ermittelt und mit der momentanen Prüfrichtung 13' der Gegeneinanderbewegung von Druckkolben und Gegenlager verglichen. Druckkolben und Gegenlager werden durch die Drehung des Haltebügels 8' derart um eine senkrecht zur Tischebene verlaufende Drehachse gedreht, daß die Richtungen 12' und 13' übereinstimmen.

Der Zustand der Vorrichtung nach dieser Drehung ist in Figur 4 dargestellt. Hier ist der Haltebügel 8' mitsamt Druckkolben 3' und Gegenlager 4' derart ausgerichtet, daß die momentane Prüfrichtung 13' nunmehr mit der Vorzugsachse 12' des Prüfkörpers 7' übereinstimmt. Der Prüfkörper 7' ist dabei in seiner ursprünglichen Position unverändert auf dem Tisch 1' liegengeblieben. In einem zweiten Arbeitsschritt wird nun der Haltebügel 8' derart in Prüfrichtung 13' verschoben, daß die Frontfläche 14' des Gegenlagers 4' ganz an den Prüfkörper 7' herangeschoben ist. Da der ganze Haltebügel 8' parallel zur Tischfläche verschoben wird, bleibt dabei der Abstand zwischen Druckkolben und Gegenlager konstant. Diese herangeschobene Position ist gestrichelt dargestellt. Im herangeschobenen Zustand berührt die Frontfläche 14' des Gegenlagers 4' den Prüfkörper 7', bewegt ihn jedoch nicht.

Im darauffolgenden Arbeitsschritt wird die Position des Haltebügels im Raum fest gelassen und der Druckkolben 3' in Prüfrichtung 13', welche inzwischen mit der Vorzugsachse 12' des Prüfkörpers 7' übereinstimmt, gegen das Gegenlager 4' geschoben. Dies ist schematisch in Figur 4 dargestellt.

Figur 4 zeigt den in seiner Position unverändert gebliebenen Prüfkörper 7' auf dem Prüftisch 1. Das Gegenlager 4' wurde durch Bewegung des Haltebügels 8' an den Prüfkörper 7' herangeschoben. Nun wird der Druckkolben 3' mit dem ihm zugeordneten Steuerelement 6' an den Prüfkörper 7' herangeschoben. Diese Position, in welcher nunmehr die Härte gemessen werden kann, ist im Gegensatz zur ursprünglichen Position des Druckkolben 3' gestrichelt dargestellt.

Bei der Härtemessung drückt der Druckkolben 3' den Prüfkörper 7' in Prüfrichtung 13' auf das Gegenlager 4'. Der Druck auf dem Prüfkörper 7' wird schrittweise erhöht, bis der Prüfkörper 7' zerbricht. Der kurz vor dem Zerbrechen gemessene maximale Druck bzw. die entsprechende vom Steuerelement 6' oder dem Druckkolben 3' ausgeübte Kraft wird gespeichert und dient als Maß für die Härte des Prüfkörpers. Die dazugehörige Kraftmeßdose befindet sich aus statischen Gründen vorzugsweise innerhalb des Gegenlagers, da die an der anderen Seite des Haltebügels angebrachte Motorsteuerung zur Bewegung des Druckkolbens im allgemeinen bereits sehr schwer ist.

Nach dem Heranfahren des Gegenlagers 4' an den Prüfkörper 7' wird damit die Härte der Tablette bzw. des Prüfkörpers in bekannter Weise gemessen.

Nach dem Zerbrechen des Prüfkörpers werden Druckkolben und Gegenlager vorzugsweise in ihrer Ausgangsposition zurückbewegt, wie in Figur 3 dargestellt. Die Bruchstücke der Tablette können nun entfernt werden und ein neuer Prüfkörper kann auch den Prüftisch befördert werden.

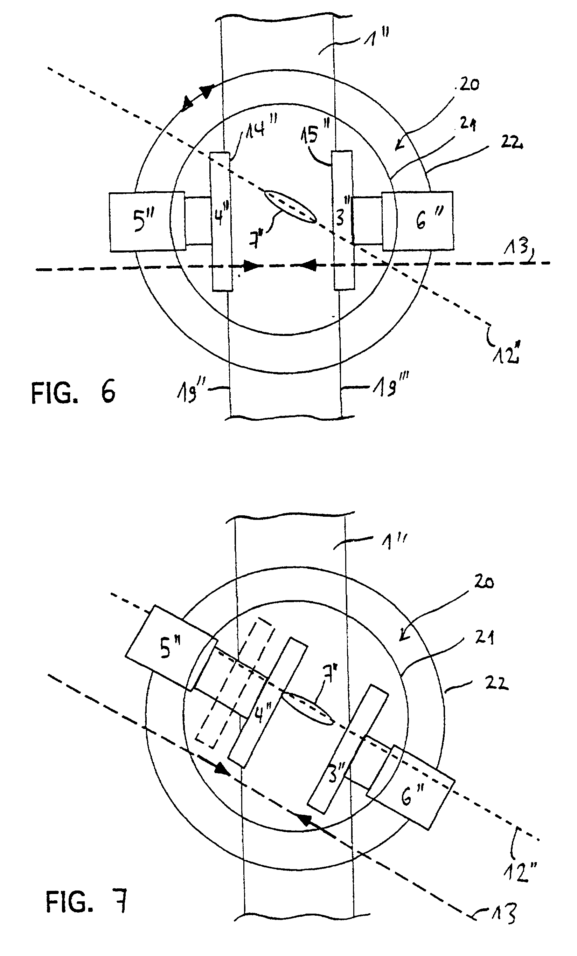

Die Figuren 6 und 7 zeigen schematisch eine Aufsicht auf ein weiteres Beispiel einer erfindungsgemäßen Vorrichtung. Der hier dargestellte Härtetester weist die aus den voranstehenden Figuren bekannten Elemente Prüftisch 1", Druckkolben 3" und Gegenlager 4" sowie das zum Druckkolben gehörige Steuerelemente 6" auf. Im Gegensatz zur Vorrichtung aus den vorangegegangenen Figuren sind hier Druckkolben 3" und Gegenlager 4" unabhängig voneinander in Prüfrichtung 13 beweglich, wobei das Gegenlager wie der Druckkolben mit einem Steuerelement 5" bewegt wird.

Figur 6 zeigt die Ausgangsposition der Anordnung vor der Härtemessung. Auf dem Prüftisch 1" befindet sich ein Prüfkörper 7" im Bereich zwischen Druckkolben 3" und Gegenlager 4". Er wurde entweder von oben oder seitlich in die Meßstelle hineinbefördert, z.B. mittels eines hier nicht dargestellten Schiebers. Zur seitlichen Zufuhr von Prüfkörpern kann der Prüftisch 1" als längliches Band oder auch als Rad ausgeführt sein, welches von einer vorhergehenden Meßstelle, z.B. Gewichtsmessung, zur Härtemeßstelle führt.

In der Ausgangs- und Ruheposition von Druckkolben 3" und Gegenlager 4" sind die jeweiligen Frontflächen 14", 15" parallel zu den Seiten 19", 19"' des Prüftisches 1". Vorzugsweise sind Druckkolben 3" und Gegenlager 4", wie hier dargestellt, so angeordnet, daß der Abstand ihrer Frontflächen 14", 15" geringer als die Breite des Prüftisches ist. Dadurch bilden die Frontflächen 14", 15" eine seitliche Begrenzung des Prüftisches und verhindern ein ungewolltes Herausfallen des Prüfkörpers aus der Meßstelle.

Die Bewegung von Druckkolben 3" und Gegenlager 4" erfolgt ähnlich wie in den Figuren 3 bis 3 dargestellt, d.h. zunächst Anpassung der Prüfrichtung 13 an die Vorzugsachse 12" und Heranschieben des Gegenlagers 4' -hier unabhängig von einem Haltebügel und damit vom Druckkolben - an den Prüfkörper 7", daraufhin Durchführung des Härtetests durch Gegendruck des Druckkolbens in Prüfrichtung gegen den Prüfkörper. Diese einzelnen Arbeitsschritte sind schematisch in den Firguren 6 und 7 gezeigt.

In der hier dargestellten Ausführung der Erfindung ist die Drehbewegung von Druckkolben 3" und Gegenlager 4" um eine Achse senkrecht zur Prüftischebene mit einer Ringkonstruktion 20, bestehend aus einem inneren und einem äußeren Ring 21 bzw. 22, realisiert. Druckkolben und Gegenlager sind mittels der Ringkonstruktion gehaltert, dabei sind ihre Steuerelemente 5", 6" mit dem äußeren Ring 22 fest verbunden. Es wird zum Ausrichten der Prüfrichtung stets der gesamte äußere Ring 22 gedreht, der dazu beispielsweise als Zahnrad ausgeführt sein kann, welches motorbetrieben genau kontrolliert um definierte Winkel gedreht werden kann. Durch die feste Halterung im äußeren Ring 22 ist sichergestellt, daß Druckkolben 3" und Gegenlager 4" einander stets diametral gegenüberliegen und ihre Frontflächen 14", 15" stets parallel zueinander sind.

Der innere Ring 21 weist eine Schiene oder einen Schlitz zur gleitenden Führung von Druckkolben 3" und Gegenlager 4" bzw. der entsprechenden Steuerelemente 5", 6" auf. Im Gegensatz zum äußeren Ring 22 bleibt er ortsfest und dient zum Stützen der Preßvorrichtung (Druckkolben 3" und Gegenlager 4" mit Steuerelementen 5", 6"). Er kann als Zylinder ausgebildet sein.

Die Ringkonstruktion 20 ist so gestaltet, daß im Bereich des Prüftisches 1" die Zu- und Abfuhr von Prüfkörpern bzw. den Bruchstücken problemlos möglich ist, z.B. weist der innere Ring 21 im Bereich des Tisches Öffungen auf, und der äußere Ring 22 verläuft in größerem Abstand zum Prüftisch.

Falls Druckkolben 3" und Gegenlager 4" etwa die Breite des Meßstelle bzw. die Breite des Prüftisches haben, kann auf eine translatorische Bewegung der Einheit aus Druckkolben 3' und Gegenlager 4' durch Bewegung des Ringes parallel zum Prüftisch 1" verzichtet werden. Alle Prüfkörper im Bereich der Meßstelle können dann durch eine reine Drehbewegung von Druckkolben 3" und Gegenlager 4" mit anschließendem Heranfahren des Gegenlagers an den Prüfkörper in Richtung ihrer Vorzugsachse zerdrückt werden. Dazu sind allerdings Verkippungen von Druckkolben 3" und Gegenlager 4" gegen ihre jeweiligen Steuerelemente 6' bzw. 5' durch die Hebelwirkung beim Zerdrücken eines nicht mittig auf die Frontflächen treffenden Prüfkörpers zu vermeiden.

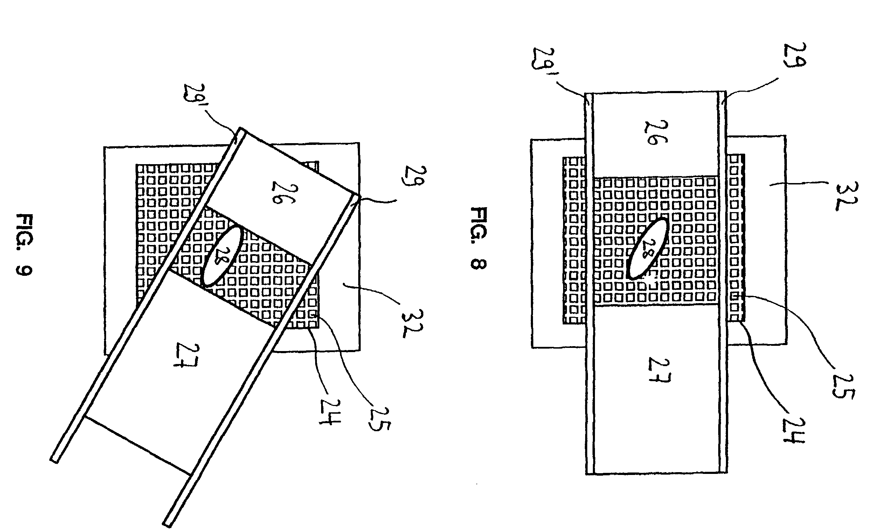

Die Figuren 8 und 9 zeigen eine Aufsicht auf den Prüftisch 32 einer Härtetestvorrichtung, welche eine Sensormatrix 24 als Vorrichtung zur Lageerkennung des Prüfkörpers aufweist. Die Matrix 24 besteht aus einer Mehrzahl von regelmäßig angeordneten optischen Sensorelementen 25, welche in den Prüftisch 32 eingebracht sind. Zum Schutz der Sensorelemente 25 ist die Matrix mit einem optisch transparenten Material abgedeckt, wobei die Oberseite des Prüftisches glatt und möglichst reibungsarm ist.

Durch geeignete Auswertung der Meßsignale der Matrix 25 wird die Lage eines Prüfkörpers 28 auf dem Prüftisch bestimmt. Die Meßsignale werden z.B. von den optischen Sensoren erzeugt, wenn ein Schattenwurf auf die aktive Fläche des Sensorelements erfolgt. Durch Zuordnung der Signale zur räumlichen Lage der Sensoren gelingt die Rekonstruktion der Position und der Kontur des Prüfkörpers, damit kann auch dessen Vorzugsrichtung, entlang derer die Härte geprüft werden soll, ermittelt werden.

Weiterhin ermöglicht die Matrix 25 die Ermittlung der Position von Druckkolben 26 und Gegenlager 27, da sich diese ebenfalls oberhalb des von der Matrix abgedeckten Bereichs befinden. Damit können Korrekturdaten zur Anpassung der Position von Druckkolben 26 und Gegenlager 27 an die Lage der Tablette durch Vergleich der jeweiligen räumlichen Positionen ermittelt werden. Ebenso kann die Anpassung der Positionen in einem iterativen Prozeß durch wiederholte Auswertung der Ausgangssignale der Sensormatrix erfolgen.

Figur 8 zeigt die Ausgangsposition bei Beginn des Härtetests. Der Prüfkörper 28 befindet sich auf dem Prüftisch im Bereich zwischen Druckkolben 26 und Gegenlager 27, welche über zwei Schienen 29 und 29', die als Haltebügel fungieren, miteinander verbunden sind. In Figur 9 ist bereits die Anpassung der Positionen von Druckkolben 26 und Gegenlager 27 an die Lage der Tablette 28, die gegenüber Figur 8 unverändert ist, durchgeführt. Die Frontfläche des Gegenlagers 27 befindet sich in unmittelbarer räumlicher Nähe des Prüfkörpers 28, berührt diesen aber zunächst nicht, da dadurch dessen Lage verändert werden könnte und erneut eine Positionsanpassung durchgeführt werden müßte. In Figur 9 ist der Druckkolben 26 ebenfalls bis in die unmittelbare Nähe der Tablette herangefahren worden. Die Steuerelemente zur Bewegung des Druckkolbens befinden sich bei der hier gezeigten Vorrichtung im Bereich des Gegenlagers 27, wobei die Kraftübertragung über die Schienen 29, 29' erfolgt, die fest mit dem Druckkolben 26 verbunden sind und diesen bei der Härteprüfung an das Gegenlager 27 heranziehen. Die Prüfrichtung ist durch die Ausrichtung der Schienen 29, 29' vorgegeben.

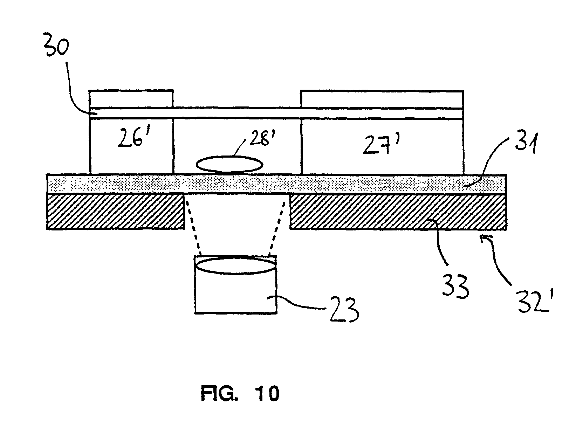

Figur 10 zeigt in Seitenansicht eine Härtetestvorrichtung mit teilweise transparentem Prüftisch 32', bestehend aus einer transparenten Platte 31 und einem Untertisch 33, wobei unterhalb desselben eine Kamera 23 angeordnet ist. Auf dem Prüftisch 32' befindet sich ein Prüfkörper 28' in der Meßstelle zwischen Druckkolben 26' und Gegenlager 27'. Im Bereich unterhalb der Meßstelle befindet sich eine Aussparung im Untertisch 33, so daß der Prüfkörper durch die transparente Platte 33 für die Kamera 23 sichtbar ist. Damit kann durch Auswertung der Kamerasignale die Information über die momentanen Positionen von Prüfkörper 28', Druckkolben 26' und Gegenlager 27' gewonnen werden.

Die transparente Platte 31 besteht aus glattem, möglichst kratzfestem und reibungsarmen Material, vorzugsweise Glas oder Acrylglas. Die Kamera 23 ist eine gewöhnliche Videokamera oder eine CCD-Kamera. Druckkolben 26' und Gegenlager 27' sind über einen Haltebügel 30, z.B. gebildet durch zwei Schienen, miteinander verbunden. Mittels des Haltebügels 30 und im Bereich des Gegenlagers angeordneter Steuerelemente, z.B. Schrittmotoren, wird der Druckkolben an das Gegenlager herangezogen. Die Registrierung und gegebenenfalls Auswertung der Signale der Kraftmeßdose findet ebenfalls im Bereich des Gegenlagers statt.

### Gewerbliche Anwendbarkeit:

Die Erfindung ist insbesondere im Rahmen der Qualitätskontrolle bei der Fertigung von Arzneiformkörpern aller Art gewerblich anwendbar.

### Liste der Bezugszeichen:

- 1, 1', 1",32, 32': Prüftisch
- 2, 33: Untertisch
- 3, 3', 3",26,26': Druckkolben
- 4, 4', 4",27,27': Gegenlager
- 5", 6, 6', 6": Steuerelement
- 7, 7', 7",28,28': Prüfkörper (Tablette)
- 8, 8', 30: Haltebügel
- 9: Haltearm
- 10: Vorrichtung zur Lagebestimmung
- 11: Drehachse
- 12, 12', 12": Vorzugsachse
- 13, 13': Prüfrichtung
- 14,14',14", 15,15', 15": Frontfläche (Druckkolben bzw. Gegenlager)
- 16: Tischfläche (Prüftisch)
- 17,18: Unterseite (Druckkolben bzw. Gegenlager)
- 19, 19': Seiten (Prüftisch)
- 20: Ringkonstruktion
- 21: innerer Ring
- 22: äußerer Ring
- 23: Kamera
- 24: Matrix
- 25: Sensorelement
- 29,29': Schiene
- 31: transparente Platte

## Patentansprüche

1. Verfahren zur Durchführung eines Härtetests an Prüfkörpern (7, 7', 7", 28, 28'), insbesondere Tabletten oder Pillen, wobei der Prüfkörper auf einem Prüftisch (1, 1', 1", 32, 32') zwischen einen Druckkolben (3,3',3",26,26') und ein Gegenlager (4,4',4",27,27') befördert und die Härte des Prüfkörpers durch das Gegeneinanderdrücken von Druckkolben (3,3',3",26,26') und Gegenlager (4,4',4",27,27') gemessen wird, **dadurch gekennzeichnet**,
a) daß auf optischem, elektronischem oder akustischem Weg die räumliche Lage einer Vorzugsachse (12, 12', 12") des Prüfkörpers (7, 7', 7", 28, 28') ermittelt wird;
b) daß Korrekturdaten ermittelt werden, die von diesen Lagedaten und von der Position von Druckkolben und Gegenlager abhängig sind;
c) daß Druckkolben (3,3',3",26,26') und Gegenlager (4,4',4",27,27') aufgrund dieser Korrekturdaten so relativ zum in seiner Lage auf dem Prüftisch unveränderten Prüfkörper verfahren werden, daß die Prüfrichtung (13), d.h. die Richtung des Gegeneinanderdrückens von Druckkolben (3,3',3",26,26') und Gegenlager (4,4',4",27,27'), mit der Richtung der Vorzugsachse (12, 12', 12") übereinstimmt und das Gegenlager in unmittelbare Nähe des Prüfkörpers gebracht wird;
d) daß die Härte des Prüfkörpers daraufhin durch Herandrücken des Druckkolbens gegen den Prüfkörper bei unveränderter Position des Gegenlagers bestimmt wird.

2. Verfahren nach Anspruch 1, **dadurch gekennzeichnet,**
**daß** die Lageerkennung auf optischem, elektronischem oder akustischem Weg erfolgt, wobei die dabei gemessenen Signale in ein Bild der Meßstelle umgesetzt werden, anhand dessen durch Bildverarbeitung die Korrekturdaten ermittelt werden.

3. Verfahren nach Anspruch 1 oder 2, **dadurch gekennzeichnet,**
**daß** mehrmals während Anpassung der Lage von Druckkolben (3,3',3",26,26') und Gegenlager (4,4',4",27,27') an die Lage des Prüfkörpers Korrekturdaten zur Steuerung des Anpassungsprozesses ermittelt werden und die Anpassung iterativ durchgeführt wird.

4. Verfahren nach Anspruch 1, **dadurch gekennzeichnet,**
**daß** der Winkel zwischen der Vorzugsachse (12, 12', 12") des Prüfkörpers (7, 7', 7", 28, 28') und der Prüfrichtung (13) ermittelt, Druckkolben (3,3',3",26,26') und Gegenlager (4,4',4",27,27') gemeinsam um diesen Winkel gedreht werden, anschließend der Abstand des Gegenlagers (4,4',4",27,27') zum Prüfkörper ermittelt und das Gegenlager (4,4',4",27,27') um diesen Abstand derart verfahren wird, daß es sich in unmittelbarer Nähe des Prüfkörpers befindet, und daraufhin der Härtetest ausgeführt wird.

5. Vorrichtung zur Durchführung eines Härtetests an Prüfkörpern (7, 7', 7", 28, 28'), insbesondere zur Durchführung des Verfahren nach einem der Ansprüche 1 bis 4, mit einem Prüftisch (1, 1', 1", 32, 32') zur Aufnahme des Prüfkörpers (7, 7', 7", 28, 28') sowie einem Druckkolben (3,3',3",26,26') und einem Gegenlager (4,4',4",27,27'), die oberhalb des Prüftischs (1, 1', 1", 32, 32') angeordnet und gegeneinander verfahrbar sind, wobei sich der Prüfkörper (7, 7', 7", 28, 28') im Anwendungsfall zwischen Druckkolben (3,3',3",26,26') und Gegenlager (4,4',4",27,27') befindet und die beim Gegeneinanderdrücken von Druckkolben (3,3',3",26,26') und Gegenlager (4,4',4",27,27') aufgewendete Kraft oder eine dazu proportionale Größe mittels einer Kraftmeßvorrichtung meßbar und die Härte des Prüfkörpers daraus bestimmbar ist, **gekennzeichnet durch** folgende Merkmale:
a) wenigstens ein Steuerelement (5", 6, 6', 6"), mit welchem Druckkolben (3,3',3",26,26') und Gegenlager (4,4',4",27,27') in der Ebene des Prüftischs in zwei Raumrichtungen verfahrbar sind;
b) eine Vorrichtung zur Lageerkennung (10) des auf dem Prüftisch (1, 1', 1", 32, 32') liegenden Prüfkörpers (7, 7', 7", 28, 28'), welche ein Bild der Meßstelle auf optischem, elektronischem oder akustischem Weg aufzuzeichnen und aus der räumlichen Lage von Druckkolben (3,3',3",26,26') und Gegenlager (4,4',4",27,27') sowie einer Vorzugsachse (12, 12', 12") des Prüfkörpers (7, 7', 7", 28, 28') Korrekturdaten zu ermitteln imstande ist, die als Ausgangssignal abgegeben werden;
c) das Ausgangssignal der Vorrichtung zur Lageerkennung (10) dient zur Ansteuerung des Steuerelements (5", 6, 6', 6") bzw. der Steuerelemente derart, daß die Prüfrichtung (13), d.h. die Richtung des Gegeneinanderdrückens von Druckkolben (3,3',3",26,26') und Gegenlager (4,4',4",27,27'), mit der Richtung der im Raum festliegenden Vorzugsachse (12, 12', 12") des Prüfkörpers (7, 7', 7", 28, 28') in Übereinstimmung gebracht wird.

6. Vorrichtung nach Anspruch 5, **dadurch gekennzeichnet,**
**daß** die Vorrichtung zur Lageerkennung (10) einen Bildverarbeitungsmechanismus enthält, insbesondere mit einer bildverarbeitungsfähigen Datenverarbeitungsanlage gekoppelt ist.

7. Vorrichtung nach Anspruch 5 oder 6, **dadurch gekennzeichnet,**
**daß** Druckkolben (3,3',3",26,26') und Gegenlager (4,4',4",27,27') über einen Haltebügel (8, 8', 30) derart miteinander verbunden sind, daß der Abstand ihrer jeweiligen Frontflächen (14, 14',14" bzw. 15, 15',15") veränderlich ist, und der Haltebügel (8) um eine senkrecht zum Prüftisch (1, 1', 1", 32, 32') verlaufende Drehachse (11) mindestens um nahezu 180 Grad drehbar ist, wobei der Haltebügel (8,8', 30) vorzugsweise mit einem Haltearm (9) oberhalb des Prüftischs gehaltert ist.

8. Vorrichtung nach Anspruch 5, **dadurch gekennzeichnet,**
**daß** Druckkolben (3,3',3",26,26') und Gegenlager (4,4',4",27,27') an einem parallel zur Tischebene um die Meßstelle umlaufenden Ring (20, 21, 22) einander gegenüberliegend gehaltert sind und der Abstand ihrer jeweiligen Frontflächen (14, 14',14" bzw. 15, 15',15") veränderlich ist.

9. Vorrichtung nach Anspruch 8, **dadurch gekennzeichnet,**
**daß** zum Einstellen der Prüfrichtung der gesamte Ring (20, 21, 22) drehbar ist oder Druckkolben (3,3',3",26,26') und Gegenlager (4,4',4",27,27') innerhalb des Ringes (20, 21, 22) oder geführt durch den Ring (20, 21, 22) drehbar sind.

10. Vorrichtung nach Anspruch 8 oder 9, **dadurch gekennzeichnet,**
**daß** der Ring seitliche Aussparungen zum Zu- und Abführen von Prüfkörpern bzw. Bruchstücken von Prüfkörpern aufweist.

11. Vorrichtung nach Anspruch 7 oder 8, **dadurch gekennzeichnet,**
**daß** der Haltebügel (8,8') bzw. der Ring (20, 21, 22) senkrecht zum Prüftisch beweglich ist.

12. Vorrichtung nach Anspruch 7 oder 8, **dadurch gekennzeichnet,**
**daß** Druckkolben (3,3',3",26,26') und/oder Gegenlager (4,4',4",27,27') in Prüfrichtung mit Schrittmotoren relativ zum Haltebügel (8,8',30) bzw. zum Ring (20, 21, 22) beweglich sind.

13. Vorrichtung nach Anspruch 7, **dadurch gekennzeichnet,**
**daß** das Gegenlager (4,4',4",27,27') fest mit dem Haltebügel (8, 8', 30) verbunden ist, wobei der Druckkolben (3,3',3",26,26') mit einem Schrittmotor gegen das Gegenlager beweglich ist.

14. Vorrichtung nach einem der vorangegangenen Ansprüche,**dadurch gekennzeichnet,**
**daß** der Prüftisch (1, 1', 1", 32, 32') eine Markierung, insbesondere ein Raster, zur Vereinfachung der Auswertung der Signale der Vorrichtung zur Lagebstimmung (10) im Hinblick auf die Bestimmung der Position und der Vorzugsachse (12, 12', 12") des Prüfkörpers (7, 7', 7", 28, 28') aufweist.

15. Vorrichtung nach einem der vorangegangenen Ansprüche, **dadurch gekennzeichnet,**
**daß** die Vorrichtung zur Lagebstimmung (10) eine Kamera (23), insbesondere eine CCD-Kamera, ist, welche vorzugsweise mit einer Datenverarbeitungsanlage gekoppelt ist.

16. Vorrichtung nach Anspruch 15, **dadurch gekennzeichnet,**
**daß** der Prüftisch wenigstens teilweise optisch transparent ist, wobei die Kamera (23) zur Lageerkennung des Prüfkörpers unterhalb des Prüftisches angeordnet ist.

17. Vorrichtung nach einem der Ansprüche 5 bis 14, **dadurch gekennzeichnet,**
**daß** die Vorrichtung zur Lagebestimmung (10) eine Einrichtung zur Abtastung eines Objekts, hier des Prüfkörpers, mittels Laserstrahlen ist, welche vorzugsweise mit einer Datenverarbeitungsanlage gekoppelt ist.

18. Vorrichtung nach einem der Ansprüche 5 bis 14, **dadurch gekennzeichnet,**
**daß** die Vorrichtung zur Lagebestimmung (10) eine vorzugsweise mit einer Datenverarbeitungsanlage gekoppelte Matrix (24) aus optischen oder Berührungssensoren (25) ist, die in den Prüftisch wenigstens im Bereich der Meßstelle eingebracht ist und Konturen auf dem Prüftisch liegender Prüfkörper zu erkennen imstande ist.

19. Vorrichtung nach Anspruch 18, **dadurch gekennzeichnet,**
**daß** die Matrix eine CCD-Matrix oder ein Diodenarray ist.

20. Vorrichtung nach einem der Ansprüche 5 bis 13, **dadurch gekennzeichnet,**
**daß** die Lage des Prüfkörpers akustisch mittels Ultraschall ermittelt wird.

## Claims

1. Process for conducting a hardness test on test specimens (7, 7', 7", 28, 28'), especially tablets or pills, wherein the test specimen is conveyed on a testing table (1, 1', 1", 32, 32') between a pressure piston (3, 3', 3", 26, 26') and a thrust bearing (4, 4', 4", 27, 27') and the hardness of the test specimen is measured by pressing the pressure piston (3, 3', 3", 26, 26') and the thrust bearing (4, 4', 4", 27, 27') against each other, **characterized in that**,
a) in an optical, electronic or acoustical manner, the spatial position of a preferred axis (12, 12', 12") of the test specimen (7, 7', 7", 28, 28') is determined;
b) correction data are determined, which are dependent on these positional data as well as on the position of the pressure piston and the thrust bearing;
c) on the basis of these correction data, the pressure piston (3, 3', 3", 26, 26') and the thrust bearing (4, 4', 4", 27, 27') are moved relative to the test specimen, whose position is unchanged on the testing table, in such a way that the test direction (13), that is to say, the direction in which the pressure piston (3, 3', 3", 26, 26') and the thrust bearing (4, 4', 4", 27, 27') press against each other, coincides with the direction of the preferred axis (12, 12', 12") and the thrust bearing is brought into the immediate proximity of the test specimen;
d) the hardness of the test specimen is then determined in the known way by pressing the pressure piston against the test specimen, while the position of the thrust bearing remains unchanged.

2. Process according to Claim 1, **characterized in that**
the position is detected in an optical, electronic or acoustical manner, whereby the measured signals are converted into an image of the measurement site, on the basis of which the correction data are determined by means of image processing.

3. Process according to Claim 1 or 2, **characterized in that**
several times during the adjustment of the position of the pressure piston (3, 3', 3", 26, 26') and the thrust bearing (4, 4', 4", 27, 27') to the position of the test specimen, correction data are determined for purposes of controlling the adjustment process and the adjustment is carried out iteratively.

4. Process according to Claim 1, **characterized in that**
the angle between the preferred axis (12, 12', 12") of the test specimen (7, 7', 7", 28, 28') and the test direction (13) is determined, the pressure piston (3, 3', 3", 26, 26') and the thrust bearing (4, 4', 4", 27, 27') are rotated jointly by this angle, subsequently, the distance of the thrust bearing (4, 4', 4", 27, 27') from the test specimen is determined and the thrust bearing (4, 4', 4", 27, 27') is moved by this distance in such a way that it is situated in the immediate proximity of the test specimen and then the hardness test is conducted.

5. Device for conducting a hardness test on test specimens (7, 7', 7", 28, 28'), especially for conducting the process according to one of Claims 1 through 4, having a testing table (1, 1', 1", 32, 32') for holding the test specimen (7, 7', 7", 28, 28') as well as a pressure piston (3, 3', 3", 26, 26') and a thrust bearing (4, 4', 4", 27, 27'), which are arranged above the testing table (1, 1', 1", 32, 32') and can move toward each other, wherein the test specimen (7, 7', 7", 28, 28') is situated between the pressure piston (3, 3', 3", 26, 26') and the thrust bearing (4, 4', 4", 27, 27') when this device is in use, and the force employed in pressing the pressure piston (3, 3', 3", 26, 26') and the thrust bearing (4, 4', 4", 27, 27') against each other or a quantity proportional to it can be measured by means of a dynamometer device and the hardness of the test specimen can be determined from this, **characterized by** the following features:
a) at least one control element (5", 6, 6', 6") with which the pressure piston (3, 3', 3", 26, 26') and the thrust bearing (4, 4', 4", 27, 27') can move in the plane of the testing table in two spatial directions;
b) a position-detecting device (10) of the test specimen (7, 7', 7", 28, 28') lying on the testing table (1, 1', 1", 32, 32'), which, in an optical or acoustical manner, is capable of recording an image of the measurement site and of using the spatial position of the pressure piston (3, 3', 3", 26, 26') and the thrust bearing (4, 4', 4", 27, 27') as well as a preferred axis (12, 12', 12") of the test specimen (7, 7', 7", 28, 28') to determine correction data, which are emitted as an output signal;
c) the output signal of the position-detecting device (10) serves to actuate the control element (5", 6, 6', 6") or the control elements in such a way that the test direction (13), i.e. the direction of movement of the pressure piston (3, 3', 3", 26, 26') and the thrust bearing (4, 4', 4", 27, 27') against each other, is brought to coincide with the direction of the spatially fixed preferred axis (12,12', 12") of the test specimen (7, 7', 7", 28, 28').

6. Device according to Claim 5, **characterized in that**
the position-detecting device (10) comprises an image processing mechanism, especially it is coupled to a data processing system that is capable of processing images.

7. Device according to Claim 5 or 6, **characterized in that**
the pressure piston (3, 3', 3", 26, 26') and the thrust bearing (4, 4', 4", 27, 27') are joined to each other by means of a holding bracket (8, 8', 30) in such a way that the distance of their respective front surfaces (14, 14', 14" or 15, 15', 15") is adjustable and the holding bracket (8) can be rotated by at least an angle of nearly 180° around an axis of rotation (11) that runs perpendicular to the testing table (1, 1', 1", 32, 32'), whereby the holding bracket (8, 8', 30) is held above the testing table, preferably with a holding arm (9).

8. Device according to Claim 5, **characterized in that**
the pressure piston (3, 3', 3", 26, 26') and the thrust bearing (4, 4', 4", 27, 27') are attached across from each other to a ring (20, 21, 22) that runs around the measurement site, parallel to the plane of the table, and the distance between their respective front surfaces (14, 14', 14" or 15, 15', 15") can be changed.

9. Device according to Claim 8, **characterized in that**
in order to adjust the test direction, the entire ring (20, 21, 22) can be rotated or the pressure piston (3, 3', 3", 26, 26') and the thrust bearing (4, 4', 4", 27, 27') can be rotated within the ring (20, 21, 22) or guided by the ring (20, 21, 22).

10. Device according to Claim 8 or 9, **characterized in that**
the ring has lateral recesses to feed test specimens or remove fragments of test specimens.

11. Device according to Claim 7 or 8, **characterized in that**
the holding bracket (8, 8') or the ring (20, 21, 22) can be moved perpendicularly to the testing table.

12. Device according to Claim 7 or 8, **characterized in that**
the pressure piston (3, 3', 3", 26, 26') and/or the thrust bearing (4, 4', 4", 27, 27') can be moved relative to the holding bracket (8, 8', 30) or relative to the ring (20, 21, 22) by means of stepping motors.

13. Device according to Claim 7, **characterized in that**
the thrust bearing (4, 4', 4", 27, 27') is firmly attached to the holding bracket (8, 8', 30), whereby the pressure piston (3, 3', 3", 26, 26') can be moved against the thrust bearing by means of a stepping motor.

14. Device according to one of the preceding claims, **characterized in that**
the testing table (1, 1', 1", 32, 32') has a marking, in particular a grid, in order to simplify the evaluation of the signals of the position-detecting device (10) for purposes of determining the position and the preferred axis (12, 12', 12") of the test specimen (7, 7', 7", 28, 28').

15. Device according to one of the preceding claims, **characterized in that**
the position-detecting device (10) is a camera (23), especially a CCD camera, which is preferably coupled to a data processing system.

16. Device according to Claim 15, **characterized in that**
the testing table is at least partially optically transparent, whereby the camera (23) for detecting the position of the test specimen is arranged beneath the testing table.

17. Device according to one of Claims 5 through 14, **characterized in that**
the position-detecting device (10) is a device for scanning an object, here the test specimen, by means of laser beams, which is preferably coupled to a data processing system.

18. Device according to one of Claims 5 through 14, **characterized in that**
the position-detecting device (10) is a matrix (24) - made up of optical or contact sensors (25) and preferably coupled to a data processing system - which is incorporated into the testing table at least in the region of the measurement site and which is capable of detecting contours of test specimens lying on the testing table.

19. Device according to Claim 18, **characterized in that**
the matrix is a CCD matrix or a diode array.

20. Device according to one of Claims 5 through 13, **characterized in that**
the position of the test specimen is determined acoustically by means of ultrasound.

## Revendications

1. Procédé pour réaliser un test de dureté sur des éprouvettes (7, 7', 7", 28, 28"), notamment des comprimés ou des pilules, l'éprouvette étant acheminée sur une table d'essai (1, 1', 1", 32, 32') entre un piston de pression (3, 3', 3", 26, 26') et une butée (4, 4', 4", 27') et la dureté de l'éprouvette étant mesurée par la pression du piston pneumatique (3, 3', 3", 26, 26') contre la butée (4, 4', 4", 27, 27'), **caractérisé en ce que**
a) la position dans l'espace d'un axe préférentiel (12, 12', 12") de l'éprouvette (7, 7', 7", 28, 28') est déterminée par voie optique, électronique ou acoustique ;
b) les données de correction déterminées dépendent desdites données de position et de la position du piston de pression (3, 3', 3", 26, 26') et de la butée (4, 4', 4", 27, 27').
c) le piston de pression (3, 3', 3", 26, 26') et la butée (4, 4', 4", 27, 27') sont déplacés du fait de ces données de correction, relativement à l'éprouvette dont la position est inchangée sur la table d'essai, de sorte que le sens de test (13), c'est-à-dire la direction dans laquelle le piston de pression (3, 3', 3", 26, 26') se comprime contre la butée (4, 4', 4", 27, 27') concorde avec la direction de l'axe préférentiel (12, 12', 12") et que la butée est amenée à proximité immédiate de l'éprouvette.
d) après quoi, la dureté de l'éprouvette est déterminée par la compression du piston de pression contre l'éprouvette, la position de la butée demeurant inchangée.

2. Procédé selon la revendication 1, **caractérisé en ce que**
la reconnaissance de position se fait par voie optique, électronique ou acoustique, les signaux mesurés à cet effet étant convertis en une image du point de mesure à l'aide de laquelle les données de correction sont déterminées par traitement de l'image.

3. Procédé selon l'une des revendications 1 ou 2, **caractérisé en ce que** à plusieurs reprises, au cours de l'adaptation de la position du piston de pression (3, 3', 3", 26, 26') et de la butée (4, 4', 4", 27, 27') à la position de l'éprouvette, les données de correction sont déterminées pour le pilotage du processus d'adaptation et **en ce que** l'adaptation se produit par itération.

4. Procédé selon la revendication 1, **caractérisé en ce que**
l'angle entre l'axe préférentiel (12, 12', 12") de l'éprouvette (7, 7', 7", 28, 28') et le sens de test (13) est déterminé, que le piston de pression (3, 3', 3", 26, 26') avec la butée (4, 4', 4", 27, 27') tournent en étant reportés de cet angle, qu'ensuite l'écart de la butée (4, 4', 4", 27, 27') à l'éprouvette est déterminé et que la butée (4, 4', 4", 27, 27') est déplacée en fonction de cet écart de manière à ce qu'elle se trouve à proximité immédiate de l'éprouvette et qu'après quoi le test de dureté est réalisé.

5. Procédé pour réaliser un test de dureté sur des éprouvettes (7, 7', 7", 28, 28'), en particulier pour réaliser le procédé selon l'une quelconque des revendications 1 à 4, avec une table d'essai (1, 1', 1", 32, 32') pour réceptionner l'éprouvette (7, 7', 7", 28, 28'), ainsi qu'un piston de pression (3, 3', 3", 26, 26') et une butée (4, 4', 4", 27, 27') qui sont placés au-dessus de la table d'essai (1, 1', 1", 32, 32') et peuvent être déplacés l'un contre l'autre, l'éprouvette (7, 7', 7", 28, 28') se trouvant dans le présent cas d'application entre un piston de pression (3, 3', 3" ; 26, 26') et une butée (4, 4', 4", 27, 27') et la force exercée lors de la compression entre le piston de pression 83, 3', 3", 26, 26") et la butée (4, 4', 4", 27, 27') ou une grandeur qui lui est proportionnelle est mesurable par un dispositif de mesure de la force et la dureté de l'éprouvette pouvant en être déduite, **caractérisé par** les attributs suivants
a) au moins un élément de pilotage (5", 6, 6', 6") avec lequel le piston de pression (3, 3', 3", 26, 26') et la butée (4, 4', 4", 27, 27') peuvent être déplacés sur le plan de la table d'essai dans deux directions dans l'espace ;
b) un dispositif pour la reconnaissance de position (10) de l'éprouvette (7, 7', 7", 28, 28') qui se trouve sur la table d'essai (1, 1', 1", 32, 32'), dispositif capable d'enregistrer une image du point de mesure par voie optique, électronique ou acoustique et de déterminer les données de correction de la position dans l'espace du piston pneumatique (3, 3', 3", 26, 26"9) et de la butée (4, 4', 4", 27, 27'), ainsi que d'un axe préférentiel (12, 12', 12") de l'éprouvette (7, 7', 7", 28, 28') qui sont transmises comme signal de sortie ;
c) le signal de sortie du dispositif pour la reconnaissance de position (10) sert au pilotage de l'élément de commande (5", 6, 6', 6"), ou des éléments de pilotage, de sorte que le sens de test (13), à savoir la direction dans laquelle le piston de pression (3, 3', 3"; 26, 26') se comprime contre la butée (4, 4', 4", 27, 27'), est amenée à concorder avec la direction de l'axe préférentiel fixe dans l'espace (12, 12', 12") de l'éprouvette (7, 7', 7", 28, 28').

6. Dispositif selon la revendication 5, **caractérisé en ce que**
le dispositif pour la reconnaissance de position (10) comporte u n mécanisme de traitement de l'image, notamment **en ce qu'**il est couplé à un système de traitement des données capable de traiter l'image.

7. Dispositif selon l'une des revendications 5 ou 6, **caractérisé en ce que**
le piston de pression (3, 3', 3", 26, 26') et la butée (4, 4', 4", 27, 27') sont reliés par un étrier de retenue (8, 8', 30), de sorte que l'écart entre leurs faces avant respectives (14, 14', 14" et 15, 15', 15") est modifiable et que l'étrier de retenue (8) peut tourner au moins de pratiquement 180 degrés autour d'un axe de rotation (11) dirigé perpendiculairement à la table d'essai (1, 1', 1", 32, 32'), l'étrier de retenue (8, 8', 30) étant de préférence soutenu par un bras de support (9) au-dessus de la table d'essai.

8. Dispositif selon la revendication 5, **caractérisé en ce que**
le piston de pression (3, 3', 3", 26, 26') et la butée (4, 4', 4", 27, 27') sont supportés en se faisant face par un anneau (20, 21, 22) qui encercle le point de mesure parallèlement au plan de la table et que l'écart entre leurs faces avant respectives (14, 14', 14" et 15, 15', 15") est modifiable.

9. Dispositif selon la revendication 8, **caractérisé en ce que**
pour l'ajustage du sens de test (13) l'anneau entier (20, 21, 22) peut tourner ou que le piston de pression (3, 3', 3", 26, 26') et la butée (4, 4', 4", 27, 27') peuvent tourner à l'intérieur de l'anneau (20, 21, 22) ou peuvent tourner guidés par l'anneau.

10. Dispositif selon l'une des revendications 8 ou 9, **caractérisé en ce que**
des réserves sont aménagées sur les côtés de l'anneau pour l'amenée et le retrait des éprouvettes ou des fragments de celles-ci.

11. Dispositif selon l'une des revendications 7 ou 8, **caractérisé en ce que**
l'étrier de retenue (8, 8') ou l'anneau (20, 21, 22) sont mobiles verticalement par rapport à la table d'essai.

12. Dispositif selon l'une des revendication 7 ou 8, **caractérisé en ce que**
le piston de pression (3, 3', 3", 26, 26') et/ou la butée (4, 4', 4", 27, 27') peuvent être mus par moteurs pas à pas dans le sens du test relativement à l'étrier de retenue (8, 8', 30) ou à l'anneau (20, 21, 22).

13. Dispositif selon la revendication 7, **caractérisé en ce que**
la butée (4, 4', 4", 27, 27') est fermement liée à l'étrier de retenue (8, 8', 30), le piston de pression (3, 3', 3", 26, 26') pouvant être mu par moteur pas à pas contre la butée.

14. Dispositif selon l'une quelconque des revendications ci-avant,
**caractérisé en ce que**
la table d'essai (1, 1', 1", 32, 32') présente un marquage, en particulier une trame destinée à simplifier l'évaluation des signaux du dispositif pour la détermination de position (10) eu égard à la détermination de la position et de l'axe préférentiel (12, 12', 12") de l'éprouvette (7, 7', 7", 28, 28').

15. Dispositif selon l'une quelconque des revendications ci-avant, **caractérisé en ce que**
le dispositif destiné à la reconnaissance de position (10) est une caméra (23), notamment une caméra CCD qui est couplée de préférence à un système de traitement des données.

16. Dispositif selon la revendication 15, **caractérisé en ce que**
la table d'essai est au moins en partie optiquement transparente, la caméra (23) pour la reconnaissance de position de l'éprouvette étant placée au-dessous de la table d'essai.

17. Dispositif selon l'une quelconque des revendications 5 à 14, **caractérisé en ce que**
le dispositif pour la reconnaissance de position (10) est une installation pour l'analyse par balayage d'un objet, ici d'une éprouvette, au moyen de rayons laser, installation qui est couplée de préférence à un système de traitement des données.

18. Dispositif selon l'une quelconque des revendications 5 à 14, **caractérisé en ce que**
le dispositif pour la détermination de position (10) est une matrice (24) de capteurs optiques ou de capteurs de contact (25) couplée de préférence avec un système de traitement de l'information qui est disposée au moins dans la zone du point de mesure et est capable de reconnaître les contours des éprouvettes sur la table d'essai.

19. Dispositif selon la revendication 18, **caractérisé en ce que**
la matrice est une matrice CCD ou une rangée de diodes.

20. Dispositif selon l'une quelconque des revendications 5 à 13, **caractérisé en ce que**
la position de l'éprouvette est déterminée par voie acoustique au moyen d'ultrasons.
